# EUROPEAN PATENT APPLICATION

(11) **EP 4 663 751 A1**
(43) Date of publication of application: **17.12.2025**
(21) Application number: 24752629.6
(22) Date of filing: 04.01.2024
(51) Int. Cl.: C12N 9/02, C12N 1/16, C12N 1/18, C12R 1/84, C12R 1/865

(54) **METHOD FOR IMPROVING PRODUCTIVITY OF BIOSYNTHETIC SUPEROXIDE DISMUTASE (SOD)**

(30) Priority: 09.02.2023 CN 202310086222
(71) Applicant: Beijing Active Blue Crystal Biotechnology Co., Ltd., Beijing 102204 (CN)
(72) Inventor: LING, Feng, Beijing 102204 (CN); DING, Wenyong, Beijing 102204 (CN); SONG, Yanxia, Beijing 102204 (CN)
(74) Representative: Mathys & Squire
(86) International application number: PCT/CN2024/070483
(87) International publication number: WO 2024/164771

(57) **Abstract**

The present invention discloses a method for increasing the productivity of biosynthetic superoxide dismutase (SOD). In the initial stage, a high yeast inoculation amount is used, combined with the addition of carbon sources, nitrogen sources and nutrients required for biosynthesis, and by increasing the dissolved oxygen value in the culture medium, using the optimal temperature and pH value, so that the yeast content in the culture medium increases rapidly in the middle stage of biosynthesis. After the middle stage of biosynthesis, the method of continuously adding carbon sources and nitrogen sources is adopted to continuously provide the biosynthetic superoxide dismutase (SOD) with the energy substances required for yeast biosynthesis. Combined with increasing the dissolved oxygen value in the culture medium and continuously adding copper and zinc salts to the fermentation tank, the yeast is stimulated to quickly synthesize copper and zinc superoxide dismutase (SOD) in the body. Through the above method, the yeast content and the superoxide dismutase (SOD) content in the yeast are increased, and the overall biosynthesis time is shortened, ultimately increasing the productivity of superoxide dismutase (SOD) biosynthesis.

## Description

### FIELD OF THE INVENTION

The present invention belongs to the field of biosynthesis technology, and more specifically relates to a method for increasing the productivity of biosynthetic superoxide dismutase (SOD).

### BACKGROUND OF THE INVENTION

Superoxide dismutase (SOD) is an important member of the antioxidant enzyme system in the biological system. It is widely present in microorganisms, plants and animals. It is an antioxidant metalloenzyme that can catalyze the dismutation of superoxide anion free radicals to generate oxygen and hydrogen peroxide. It plays a vital role in the body's oxidation and antioxidant balance and is inseparable from the occurrence and development of many diseases.

The main function of superoxide dismutase (SOD) is to catalyze the dismutation of superoxide anion free radicals into hydrogen peroxide and oxygen. Superoxide anions are the product of normal metabolism in organisms, but the accumulation of free radicals will cause lipid peroxidation of cell membranes and cause membrane fission, leading to cell damage and even death.

Superoxide dismutase (SOD) is the most important and best free radical scavenger in cells to maintain the body's metabolic balance. Superoxide dismutase is a type of metalloenzyme, which can be divided into three types:

The first type contains copper and zinc, called copper-zinc superoxide dismutase, which is blue-green and mainly exists in the cytoplasm of eukaryotic cells;

The second type contains manganese, called manganese-containing superoxide dismutase, which is purple and mainly exists in prokaryotic cells and eukaryotic cells matrix (such as mitochondria, etc.);

The third type contains iron, called iron-containing superoxide dismutase, which is yellow-brown and mainly exists in prokaryotic cells and a few plant cells.

In eukaryotic organisms, including humans, the main superoxide dismutase is the first type of copper-zinc superoxide dismutase. The superoxide dismutase biosynthesized according to the present disclosure belongs to the first type, namely copper-zinc superoxide dismutase.

Superoxide dismutase (SOD) can play an anti-oxidation and anti-aging role: Superoxide dismutase (SOD) can remove and reduce excessive free radicals in the human body and delay aging. It can continuously remove oxygen free radicals generated by excessive oxygen consumption of melanin due to light exposure, prevent the formation of excessive melanin, and also continuously remove oxygen free radicals generated by lipofuscin and waxy fat to avoid excessive lipid peroxidation, and reduce the formation of lipofuscin and waxy fat, thereby playing an anti-aging role.

Superoxide dismutase (SOD) can play an anti-tumor role: In the human body, superoxide dismutase (SOD) can effectively inhibit cancer cells by removing superoxide anion free radicals. The superoxide dismutase (SOD) in the human body tends to decline with age, Its decline leads to a decline in the body's antioxidant capacity, and then a decline in resistance. In severe cases, tumors may occur. Therefore, the elderly people are generally more likely to be patients with tumors. From this perspective, detecting the content of superoxide dismutase (SOD) in a person's body can be used as an important indicator to determine whether a person has cancer and the aging index.

Anti-inflammatory effect: Superoxide dismutase (SOD) has a strong anti-inflammatory effect. Using superoxide dismutase (SOD) to treat pleurisy can reduce the accumulation of pleural effusion and white blood cells. Superoxide dismutase (SOD) can also inhibit arthritis, reduce the precipitation of IgG in the glomeruli, and interfere with the accumulation of white blood cells in the inflammatory site of the glomeruli. Superoxide dismutase (SOD) can inhibit the changes in organ permeability caused by acute tracheitis which caused by xanthine/xanthine oxidase, and reduce the accumulation of polymorphonuclear leukocytes in the lungs. Superoxide dismutase (SOD) has a therapeutic and improving effect on idiopathic proctitis. Injecting an appropriate amount of superoxide dismutase (SOD) can improve the appearance of joints, improve joint mobility, and effectively treat osteoarthritis. It can be seen that superoxide dismutase (SOD) has a good application prospect for the treatment of inflammation.

Application fields of superoxide dismutase (SOD):

Medicine: Superoxide free radicals in the body can cause various diseases. Superoxide dismutase (SOD) is its natural scavenger and maintains a dynamic balance with it under normal circumstances. However, under pathological conditions, excessive superoxide free radicals are produced. The superoxide dismutase (SOD) produced by the body itself cannot completely remove these excessive superoxide free radicals, which are harmful to the body. Superoxide dismutase (SOD) can catalyze their dismutation reaction and alleviate the condition.

Food industry: Studies have shown that adding superoxide dismutase (SOD) to food as a natural antioxidant can be used as a preservative.

Clinical trials have shown that oral superoxide dismutase (SOD) is effective and can be absorbed into the human body through the gastrointestinal tract. Therefore, superoxide dismutase (SOD) chewing gum, superoxide dismutase (SOD) lozenges and oral liquid have appeared one after another, becoming one of the important ways to supplement superoxide dismutase (SOD) in vitro.

Daily chemical industry: The application of superoxide dismutase (SOD) in daily chemical industry is mainly skin care products and toothpaste.

Superoxide dismutase (SOD) has the following main functions in skin care products: First, as a cosmetic additive, it prevents skin aging, reduces wrinkles and removes spots, and has a skin care effect; second, it prevents and treats related skin diseases, such as dermatitis, acne, and skin burns.

Since superoxide dismutase (SOD) has an anti-inflammatory effect and is pure natural with no toxic side effects, it plays an important role in oral and dental care.

Agriculture: Overexpression of superoxide dismutase (SOD) in transgenic plants can improve the resistance of plants to adverse environments to varying degrees. By genetic engineering, increasing the expression of superoxide dismutase (SOD) in plants can greatly enhance the stress resistance of plants.

From the above introduction, it can be seen that superoxide dismutase (SOD) is important to human health and life, so it is very important to find a high-content, low-cost method for producing superoxide dismutase (SOD), especially the method for producing the first type superoxide dismutase (copper-zinc superoxide dismutase) (SOD).

There are four main methods for producing superoxide dismutase (SOD):
1. Extraction method using animal blood;
2. Extraction from plants;
3. Production by microbial biosynthesis;
4. Production of superoxide dismutase (SOD) by genetic engineering method.

Using animal blood extraction method: Due to the limitation of raw materials, it cannot be mass-produced, and there will be viruses in animal blood, which cannot be directly used for human body, and the cost is too high. Due to the above shortcomings, it cannot be industrialized and mass-produced.

Extraction from plants: Due to the limitation of raw materials and the low content of superoxide dismutase (SOD) in natural plants, it is impossible to obtain high-purity superoxide dismutase (SOD), which limits the application of superoxide dismutase (SOD) in many fields.

Production of superoxide dismutase (SOD) by genetic engineering method has only been studied in recent years. The existing technology can only produce small batches, and the cost is too high, and it cannot meet the requirements of large-scale production.

The microbial biosynthesis method is the subject of the presently disclosed biosynthesis method. The microorganisms used for the production of superoxide dismutase (SOD) by microbial biosynthesis are mainly yeast and bacteria, according to the production method of the present invention. The advantages of this method are that there is no restriction on raw materials, and the biosynthesis process can be strictly controlled to perform aseptic operation, so the produced superoxide dismutase (SOD) does not contain any harmful viruses, bacteria and other pathogens.

In the prior art, the main reason for limiting the industrial production of superoxide dismutase (SOD) biosynthesized by microorganisms (mainly yeast) is that the productivity of superoxide dismutase biosynthesized by yeast is too low, that is, the biological activity (U) of superoxide dismutase biosynthesized per unit volume of fermentation tank (liter) per unit time (hour) is too low.

In the prior art, the main problem is that at the end of biosynthesis, the yeast content in the biosynthesis liquid is too low, the superoxide dismutase (SOD) content in the yeast is too low, and the biosynthesis time is too long, that is, the productivity is too low, which makes the equipment investment too large, the raw and auxiliary materials consumption is too high, and the production cost of superoxide dismutase (SOD) is too high. In addition, since the content of biosynthesized superoxide dismutase (SOD) in yeast is too low, it causes difficulties in the later purification process and cannot obtain high-purity superoxide dismutase (SOD).

Therefore, finding a biosynthesis method with high superoxide dismutase (SOD) content, high yeast content and short time, that is, a biosynthesis method with high productivity, is the key to realizing the industrialized large-scale production of superoxide dismutase (SOD).

### SUMMARY OF THE INVENTION

The object of the present invention is to find a biosynthesis method to produce superoxide dismutase with high superoxide dismutase (SOD) content, high yeast content and short time, that is, a biosynthesis method to produce superoxide dismutase with high productivity.

The object of the present invention is achieved by the following technical scheme:

In a first embodiment, a method for increasing the productivity of biosynthetic superoxide dismutase (SOD) is provided, specifically comprising the following steps:
(1) Adding water to a fermentation tank, and then adding the carbon source, nitrogen source and nutrient salts required for yeast biosynthesis of superoxide dismutase (SOD) to obtain a basic culture medium;
(2) Using a high yeast inoculation amount in the basic culture medium;
(3) Setting the air flow rate of the fermentation tank, stirring speed, temperature and pH value, start the biosynthesis of superoxide dismutase (SOD);
(4) When the initial stage of biosynthesis is reached, start to add carbon source and nitrogen source to the fermentation tank continuously;
(5) When the middle stage of biosynthesis is reached, start to add copper and zinc salts to the fermentation tank continuously, and at the same time increase the air flow rate and stirring speed;
(6) After the biosynthesis is completed, yeast with high yeast content and high superoxide dismutase (SOD) biological activity content in the yeast is obtained in the biosynthesis liquid.

In some aspects, the yeast is selected from Saccharomyces Cerevisiae, Pichia Pastoris, etc.

In some aspects, the high yeast inoculation amount is higher than about 10g/L.

In some aspects, the carbon source described includes glucose, sucrose and/or other carbon sources.

In some aspects, the nitrogen sources include peptone, yeast powder, urea, ammonium sulfate and/or other nitrogen sources.

In some aspects, the nutrient salts include copper sulfate, zinc sulfate, dipotassium hydrogen phosphate, potassium dihydrogen phosphate, magnesium sulfate, calcium chloride, sodium chloride, and/or other nutrient salts.

In some aspects, the air flow rate is about 1-1.5 vvm (volumetric gas-toliquid ratio; i.e., air volume fed into fermentation tank per minute/biosynthetic liquid volume).

In some aspects, the stirring speed in step (3) is about 200-500 rpm.

In some aspects, the temperature is within the range of about 28-36°C.

In some aspects, the pH value is within the range of about 4.5-7.5.

In some aspects, the time of the initial stage of biosynthesis described is about 2-6 hours.

In some aspects, the carbon source and nitrogen source are added until 5-10 hours before the end of biosynthesis.

In some aspects, the copper and zinc salts include copper sulfate, zinc sulfate, and/or other coppser and zinc salts.

In some aspects, the time of the middle stage of biosynthesis is about 20-24 hours.

In some aspects, when the biosynthesis reaches the middle stage of biosynthesis, the wet yeast content in the biosynthesis liquid is not less than about 170g/L, and the dry matter content of the wet yeast is calculated as about 33%.

In some aspects, the nutrient salt is added until about 3-6 hours before the end of biosynthesis.

In some aspects, the air flow rate is about 1.5-2.5 vvm (air volume fed into the fermentation take per minute/volume of biosynthesis liquid).

In some aspects, the stirring speed in step (5) is about 500-800 rpm.

In some aspects, the biosynthesis completion time in step (6) is not more than about 55 hours.

In some aspects, the yeast content in the biosynthesis liquid in step (6) is not less than about 200 g/L, calculated as the wet yeast content per liter of biosynthesis liquid, and the dry matter content of the wet yeast is calculated as about 33%.

In some aspects, the superoxide dismutase (SOD) biological activity content in step (6) is not less than about 8000 U/g wet yeast, and the dry matter content of the wet yeast is calculated as about 33%.

In some aspects, the superoxide dismutase (SOD) biological activity in step (6) is determined by the pyrogallol auto-oxidation method.

In some aspects, the superoxide dismutase (SOD) described in step (6) is an intracellular enzyme.

The superoxide dismutase (SOD) described in step (6) is a Cu/Zn superoxide dismutase (SOD).

The advantages and beneficial effects of the present invention include:

First, the present invention belongs to a biosynthesis technology. Biosynthesis is a multi-step, enzyme-catalyzed process.

In this process, the substrate which include the carbon source, nitrogen source and nutrient salts is converted into more complex substances in the organelle. In the biosynthesis process, simple compounds are changed, converted into other compounds, or combined into macromolecular compounds. This process often includes metabolic pathways. These metabolic pathways may occur in one organelle, and some may involve several organelles. The superoxide dismutase described in the present invention is synthesized in yeast cells in this way.

Second, the present invention adopts a high yeast inoculation amount in the initial stage of biosynthesis, and the high yeast inoculation amount is combined with the addition of appropriate carbon sources, nitrogen sources and nutrients required for biosynthesis, and the dissolved oxygen value in the culture medium is increased by high air intake and high stirring speed, and the optimal temperature and pH value are adopted, so that the yeast content in the culture medium reaches 170g/L or more in the middle stage of biosynthesis.

Third, in the middle stage of biosynthesis, that is, on the basis of the yeast content in the culture medium reaching 170g/L or more, the present invention adopts a method of continuing to add carbon sources and nitrogen sources, combined with a method of increasing air flow rate and stirring speed to further increase the dissolved oxygen value in the culture medium, to provide the necessary energy substances and oxygen for the continued growth of yeast, and finally the yeast content in the biosynthesis liquid is increased, so that the yeast content in the final biosynthesis liquid exceeds 200g/L.

Fourth, in the middle stage of biosynthesis, when the content of yeast in the culture medium reaches 170 g/L or more, the present invention adopts a method of continuously adding carbon source and nitrogen source to continuously provide the energy substances required for the biosynthesis of yeast superoxide dismutase (SOD), and further increases the dissolved oxygen value in the culture medium by increasing the air flow rate and the stirring speed to provide the oxygen required for yeast biosynthesis. At the same time, by continuously adding copper and zinc salts to the fermentation tank, the yeast is stimulated to rapidly synthesize Cu/Zn superoxide dismutase (SOD) in vivo, and finally the biological activity content of superoxide dismutase (SOD) in the yeast in the biosynthesis liquid is increased, and the biological activity content of superoxide dismutase (SOD) in the yeast exceeds 8000 U/g Wet yeast.

Fifth, the present invention uses the above method to rapidly increase the yeast content in the culture medium, and the biological activity content of superoxide dismutase (SOD) in the yeast is also rapidly increased, and ultimately shortens the overall biosynthesis time, so that the biosynthesis completion time does not exceed 55 hours.

Sixth, the present invention uses a method of rapidly increasing the yeast content in the culture medium and rapidly increasing the biological activity content of superoxide dismutase (SOD) in the yeast to shorten the overall biosynthesis time, and ultimately increases the productivity of biosynthetic superoxide dismutase (SOD).

### EXAMPLES

The present invention will be further described below through specific examples.

### EXAMPLE 1

A 3-liter fully automatic fermentation tank was used, which has automatic control functions for temperature, pH and dissolved oxygen value (stirring speed and air flow rate), and has a 6-way feeding automatic control system.
(1) 70 g/L of sterilized sugar, 1 g/L of peptone, 2 g/L of yeast powder, 1.3 g/L of urea, 0.03 g/L of copper sulfate pentahydrate, 0.03 g/L of zinc sulfate heptahydrate, 1.5 g/L of dipotassium hydrogen phosphate, 3 g/L of potassium dihydrogen phosphate, and 0.3 g/L of magnesium sulfate heptahydrate were added to the fermentation tank, and then ultrapure water was added to 2.5 liters to obtain a basic culture medium.
(2) Angel brewer's yeast (fresh and dry yeast) was used, and the yeast inoculation amount was 10 g/L.
(3) The air flow rate of the fermentation tank was set to 1 v/v/m (air volume fed into the fermentation tank per minute/volume of fermentation tank), the stirring speed to 200 rpm, the temperature to 30°C and the pH value to 6.5, and the biosynthesis of superoxide dismutase (SOD) was started.
(4) After 2 hours of biosynthesis, glucose solution 7 ml/h (glucose content 50%), yeast powder solution 0.6 ml/h (yeast powder content 20%), urea solution 0.4 ml/h (urea content 20%) were added, and the addition was stopped after 45 hours.
(5) After 20 hours of biosynthesis, copper sulfate pentahydrate solution 0.01 ml/h (copper sulfate content 15%) and zinc sulfate heptahydrate solution 0.01 ml/h (zinc sulfate content 15%) were added, the air flow rate was increased to 1.5 vvm, the stirring speed was increased to 500 rpm, and the addition was stopped after 45 hours.
(6) Biosynthesis was terminated after 50 hours, and 2.6 L of biosynthesis liquid was obtained.
(7) After the biosynthesis reached 20 hours, 50 ml of the biosynthesis liquid was taken and separated with a separator. The separator was set to 6000 rpm, the separation factor was 6439, and the separation time was 20 minutes. Through separation, wet yeast is obtained. The obtained wet yeast was dissolved in 5 times the volume of water, stirred evenly and separated again. The above steps were repeated three times to obtain pure wet yeast. The weight of the pure wet yeast was 8.6 g. The content of pure wet yeast in the biosynthesis liquid was calculated to be 17.2% (w/v).
(8) 500 ml of the biosynthesis liquid after the biosynthesis is completed was taken, and the biosynthesis liquid was separated with a separator. The separator was set to 6000 rpm, the separation factor was 6439, and the separation time was 20 minutes. After separation, wet yeast was obtained. The obtained wet yeast was dissolved in 5 times the volume of water, stirred evenly and separated again. The above steps were repeated three times to obtain pure wet yeast. The weight of the pure wet yeast was 104g. The content of the pure wet yeast in the biosynthesis liquid was calculated to be 20.8% (w/v).
(9) 100g of the pure wet yeast obtained in step (8) was taken, and then a homogenizer was used to break the yeast wall. The pressure of the homogenizer was set to 140MPa, the flow rate was set to 50ml/min, and the number of cycles was 5 times to obtain a yeast wall-breaking liquid. The yeast wall-breaking liquid was tested by the pyrogallol auto-oxidation method. The superoxide dismutase (SOD) content in the wet yeast was 8200U/g

After calculation, the productivity of superoxide dismutase synthesized by yeast was 29564U/L/h { Superoxide dismutase (U) synthesized by the unit volume of fermentation tank (liter) per unit time (hour)}.

### EXAMPLE 2

A 3-liter fully automatic fermentation tank was used, which has automatic control functions for temperature, pH and dissolved oxygen value (stirring speed and air flow rate), and has a 6-way feeding automatic control system.
(1) 75 g/L of sterilized sugar, 1.2 g/L of peptone, 2.3 g/L of yeast powder, 1.2 g/L of urea, 0.04 g/L of copper sulfate pentahydrate, 0.04 g/L of zinc sulfate heptahydrate, 1.7 g/L of dipotassium hydrogen phosphate, 4 g/L of potassium dihydrogen phosphate, and 0.4 g/L of magnesium sulfate heptahydrate was added to the fermentation tank, and then ultrapure water was added to 2.5 liters to obtain a basic culture medium.
(2) Angel brewer's yeast (fresh dry yeast) was used, and the yeast inoculation amount was 12g/L.
(3) The air flow rate of the fermentation tank was set to 1.2v/v/m (air volume fed to the fermentation tank per minute/ volume of fermentation tank), the stirring speed was 300 rpm, the temperature was 31°C and the pH value was 6.8, and the biosynthesis of superoxide dismutase (SOD) was started.
(4) After 3 hours of biosynthesis, glucose solution was added at 8ml/h (glucose content was 50%), peptone solution at 0.3ml/h (peptone content was 20%), yeast powder solution at 0.6ml/h (yeast powder content was 20%), and urea solution at 0.3ml/h (urea content was 20%), and the addition was stopped after 45 hours.
(5) After 24 hours of biosynthesis, 0.02 ml/h of copper sulfate pentahydrate solution (copper sulfate content is 15%) and 0.02 ml/h of zinc sulfate heptahydrate solution (zinc sulfate content is 15%) were added, the air flow rate was increased to 2 v/v/m , the speed was increased to 700 rpm, and the addition was stopped after 46 hours.
(6) Biosynthesis was completed after 55 hours, and 2.65 L of biosynthesis liquid was obtained.
(7) After the biosynthesis reached 24 hours, 50 ml of the biosynthesis liquid was taken and separated with a separator. The separator was set to 6000 rpm, the separation factor was 6439, and the separation time was 20 minutes. Through separation, wet yeast was obtained. The obtained wet yeast was dissolved in 5 times the volume of water, stirred evenly and separated again. The above steps were repeated three times to obtain pure wet yeast. The weight of the pure wet yeast was 8.9 g. The content of pure wet yeast in the biosynthesis liquid was calculated to be 17.8% (w/v).
(8) 500 ml of the biosynthesis liquid was taken after the biosynthesis was completed, and the biosynthesis liquid was separated with a separator. The separator was set to 6000 rpm, the separation factor was 6439, and the separation time was 20 minutes. Through separation, wet yeast was obtained. The obtained wet yeast was dissolved in 5 times the volume of water, stirred evenly and separated again. The above steps were repeated three times to obtain pure wet yeast. The weight of the pure wet yeast was 112 g. The content of the pure wet yeast in the biosynthesis liquid was calculated to be 22.4% (w/v).
(9) 100g of the pure wet yeast obtained in step (8) was taken, and a homogenizer was then used to break the yeast wall. The homogenizer pressure was set to 140MPa, the flow rate was set to 50ml/min, and the number of cycles was 5 times to obtain a yeast wall-breaking liquid. The yeast wall-breaking liquid was tested by the pyrogallol auto-oxidation method. The superoxide dismutase (SOD) content in the wet yeast was 9100U/g.

After calculation, the productivity of superoxide dismutase biosynthesized by yeast was 32738U/L/h {superoxide dismutase (U) biosynthesized per unit volume of fermentation tank (liter) per unit time (hour)}.

### EXAMPLE 3

A 3-liter fully automatic fermentation tank was used, which has automatic control functions for temperature, pH and dissolved oxygen value (stirring speed and air flow rate), and has a 6-way feeding automatic control system.
(1) 80 g/L of sterilized sugar, 1.5 g/L of peptone, 2.5 g/L of yeast powder, 1.25 g/L of urea, 0.05 g/L of copper sulfate pentahydrate, 0.05 g/L of zinc sulfate heptahydrate, 2 g/L of dipotassium hydrogen phosphate, 5 g/L of potassium dihydrogen phosphate, and 0.5 g/L of magnesium sulfate heptahydrate were added to the fermentation tank, and then ultrapure water added to 2.5 liters to obtain a basic culture medium.
(2) Angel brewer's yeast (fresh and dry yeast) was used, and the yeast inoculation amount was 15 g/L.
(3) The air flow rate of the fermenter was set to 1.5v/v/m (air volume fed into the fermentation tank per minute/the volume of fermentation tank), the stirring speed to 500 rpm, the temperature to 32°C and the pH value to 7, and biosynthesis of superoxide dismutase (SOD) was started.
(4) After 4 hours of biosynthesis, started to add glucose solution 10ml/h (glucose content is 50%), peptone solution 0.4ml/h (peptone content is 20%), yeast powder solution 0.7ml/h (yeast powder content is 20%), urea solution 0.36ml/h (urea content is 20%), and stopped the addition after 48 hours.
(5) After 22 hours of biosynthesis, 0.03 ml/h of copper sulfate pentahydrate solution (copper sulfate content is 15%) and 0.03 ml/h of zinc sulfate heptahydrate solution (zinc sulfate content is 15%) were added, the air flow rate was increased to 2.5 v/v/m/, the stirring speed was increased to 800 rpm, and the addition was stopped after 50 hours.
(6) The biosynthesis was terminated after 52 hours, and 2.7 L of biosynthesis liquid was obtained.
(7) After the biosynthesis reached 22 hours, 50 ml of the biosynthesis liquid was taken and separated with a separator. The separator was set to 6000 rpm, the separation factor was 6439, and the separation time was 20 minutes. Through separation, wet yeast was obtained. The obtained wet yeast was dissolved in 5 times the volume of water, stirred evenly and separated again. The above steps were repeated three times to obtain pure wet yeast. The weight of the pure wet yeast was 9.2 g. The content of pure wet yeast in the biosynthesis liquid was calculated to be 18.4% (w/v).
(8) 500 ml of the biosynthesis liquid was taken after the biosynthesis is completed, and the biosynthesis liquid was separated with a separator. The separator was set to 6000 rpm, the separation factor was 6439, and the separation time was 20 minutes. After separation, wet yeast was obtained. The obtained wet yeast was dissolved in 5 times the volume of water, stirred evenly and separated again. The above steps were repeated three times to obtain pure wet yeast. The weight of the pure wet yeast was 118 g. The content of the pure wet yeast in the biosynthesis liquid was calculated to be 23.6% (w/v).
(9) 100g of the pure wet yeast obtained in step (8) was taken, and then a homogenizer to break the yeast cell wall. The homogenizer pressure was set to 140MPa, the flow rate was set to 50ml/min, and the number of cycles was 5 times to obtain a yeast cell wall liquid. The yeast cell wall liquid was tested by pyrogallol auto-oxidation method. The superoxide dismutase (SOD) content in the wet yeast was 9800U/g.

After calculation, the productivity of superoxide dismutase biosynthesized by yeast was 40029U/L/h {superoxide dismutase (U) biosynthesized per unit volume of fermentation tank (liter) per unit time (hour)}.

## Claims

1. A method for increasing productivity of biosynthetic superoxide dismutase (SOD), comprising the steps of:
(1) adding water to a fermenter, and then adding a carbon source, nitrogen source and nutrient salts in an amount effective for yeast biosynthesis of superoxide dismutase (SOD) to obtain a basic culture medium; and
(2) using a high yeast inoculation amount in the basic culture medium; and
(3) setting the air flow rate, stirring speed, temperature and pH value of the fermentation tank to start the biosynthesis of superoxide dismutase (SOD); and
(4) when the initial stage of biosynthesis is reached, starting to flow carbon source and nitrogen source into the fermentation tank; and
(5) when the middle stage of biosynthesis is reached, starting to flow copper and zinc salts into the fermentation tank, meanwhile, increasing the air flow rate and stirring speed; and
(6) after the biosynthesis is completed, obtaining yeast with high yeast content in the biosynthetic liquid, and high superoxide dismutase (SOD) biological activity content in the yeast.

2. The method according to claim 1, wherein the yeast is selected from Saccharomyces cerevisiae and Pichia pastoris.

3. The method according to claim 1, wherein the high yeast inoculation amount is greater than about 10 g/L.

4. The method according to claim 1, wherein the carbon source comprises glucose and/or sucrose, the nitrogen source comprises peptone, yeast powder, urea and/or ammonium sulfate, and the nutrient salts comprise copper sulfate, zinc sulfate, dipotassium hydrogen phosphate, potassium dihydrogen phosphate, magnesium sulfate, calcium chloride and/or sodium chloride.

5. The method according to claim 1, wherein, in step (3), the air flow rate is about 1-1.5 v/v/m, wherein v/v/m is the air volume fed into the fermentation tank per minute/volume of fermentation tank, the stirring speed is about 200-500 rpm, the temperature is about 28-36°C, and the pH value is about 4.5-7.5.

6. The method according to claim 1, wherein the initial stage of biosynthesis in step (4) lasts for about 2-6 hours.

7. The method according to claim 1, wherein the carbon source and nitrogen source in step (4) are added until about 5-10 hours before the end of biosynthesis.

8. The method according to claim 1, wherein the copper and zinc salts in step (5) comprise copper sulfate and/or zinc sulfate.

9. The method according to claim 1, wherein the mid-stage of biosynthesis in step (5) lasts for about 20-24 hours.

10. The method according to claim 1, wherein the middle stage of biosynthesis in step (5), is **characterized in that** when the biosynthesis reaches the middle stage of biosynthesis, the wet yeast content in the biosynthesis liquid is not less than about 170 g/L, and the dry matter content of the wet yeast is calculated as about 33%.

11. The method according to claim 1, wherein the starting to flow copper and zinc salts into the fermentation tank until about 3-6 hours before the end of biosynthesis.

12. The method according to claim 1, wherein the air flow rate is about 1.5-2.5 v/v/m and the stirring speed is about 500-800 rpm.

13. The method according to claim 1, wherein after the biosynthesis is completed in step (6), the biosynthesis is completed not exceed 55 hours.

14. The method according to claim 1, wherein the high yeast content in the biosynthetic liquid in step (6) is not less than about 200g/L, calculated according to the content of wet yeast in per liter of biosynthetic liquid, and the dry matter content of the wet yeast is calculated as about 33%.

15. The method according to claim 1, wherein the high superoxide dismutase (SOD) biological activity content in the yeast in step (6) is not less than about 8000U/g wet yeast, the dry matter content of the wet yeast is calculated as about 33%, and the superoxide dismutase (SOD) biological activity is determined by the pyrogallol auto-oxidation method.

16. The method according to claim 1, wherein the superoxide dismutase (SOD) in step (6) is an intracellular enzyme, and the superoxide dismutase (SOD) is a Cu/Zn superoxide dismutase (SOD).
